(19) Europäisches Patentamt
European Patent Office
Office européen des brevets

(11) Veröffentlichungsnummer: **0 630 947 A1**

(12) # EUROPÄISCHE PATENTANMELDUNG

(21) Anmeldenummer: **94108933.6**

(22) Anmeldetag: **10.06.94**

(51) Int. Cl.[5]: **C09B 62/507**, C09B 62/08, C07D 251/30

(30) Priorität: **22.06.93 DE 4320661**

(43) Veröffentlichungstag der Anmeldung: **28.12.94 Patentblatt 94/52**

(84) Benannte Vertragsstaaten: **CH DE FR GB IT LI**

(71) Anmelder: **BASF Aktiengesellschaft**
**Carl-Bosch-Strasse 38**
**D-67063 Ludwigshafen (DE)**

(72) Erfinder: **Wiesenfeldt, Matthias, Dr.**
**Rosenstrasse 10**
**D-67112 Mutterstadt (DE)**
Erfinder: **Siegel, Bernd, Dr.**
**Frankenstrasse 18**
**D-67166 Otterstadt (DE)**
Erfinder: **Patsch, Manfred, Dr.**
**Fritz-Wendel-Strasse 4**
**D-67157 Wachenheim (DE)**

(54) **Phenylazo- oder Naphthylazobenzole mit Thioethergruppen.**

(57) Reaktivfarbstoffe der Formel

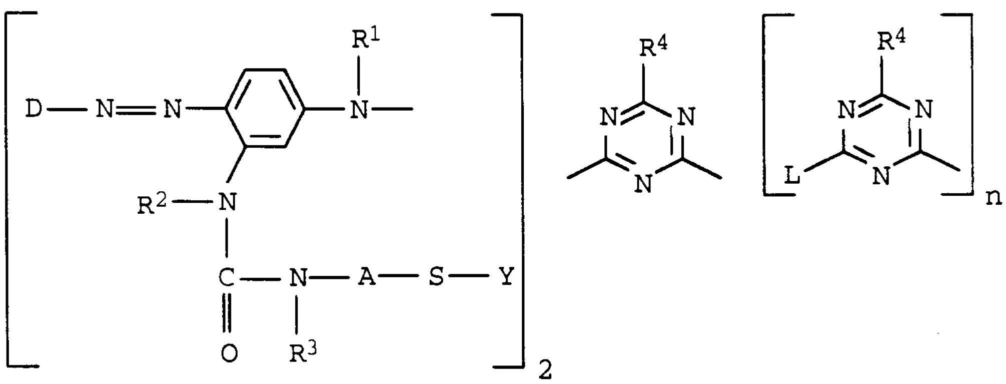

in der

| | |
|---|---|
| n | 0 oder 1 |
| $R^1$, $R^2$ und $R^3$ | jeweils Wasserstoff, $C_1$-$C_4$-Alkyl oder Phenyl, |
| $R^4$ | Fluor, Chlor, Brom, $C_1$-$C_4$-Alkylsulfonyl, Phenylsulfonyl oder einen Rest der Formel |

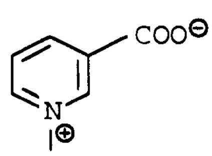

,

| | |
|---|---|
| L | ein Brückenglied, |
| A | gegebenenfalls substituiertes $C_2$-$C_8$-Alkylen, |
| Y | Vinyl oder einen Rest der Formel $-CH_2-CH_2-Q$, wobei Q für eine unter alkalischen |

EP 0 630 947 A1

Reaktionsbedingungen abspaltbare Gruppe steht, und

D Phenyl oder Naphthyl, wobei diese Reste substituiert sein können, bedeuten,

sowie ein Verfahren zur Herstellung von Reaktivfarbstoffen durch Oxidation der obengenannten Verbindungen.

Die vorliegende Erfindung betrifft neue Verbindungen der Formel I

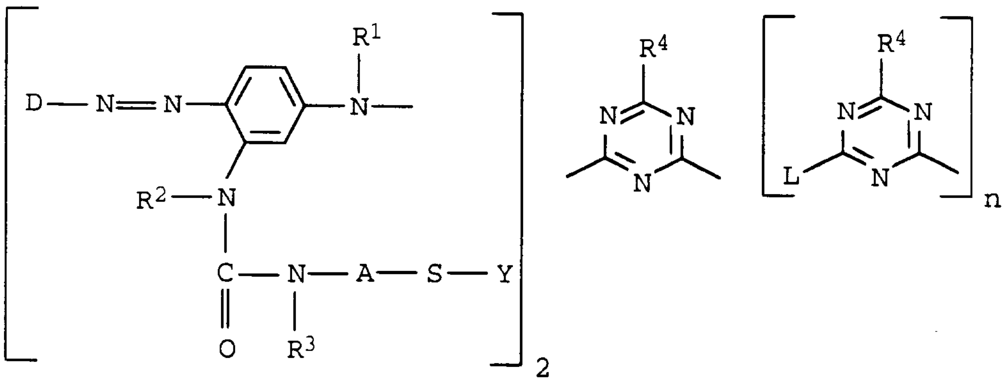

(I),

in der

n 0 oder 1

$R^1$, $R^2$ und $R^3$ gleich oder verschieden sind und unabhängig voneinander jeweils Wasserstoff, $C_1$-$C_4$-Alkyl oder Phenyl,

$R^4$ Fluor, Chlor, Brom, $C_1$-$C_4$-Alkylsulfonyl, Phenylsulfonyl oder einen Rest der Formel

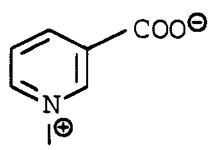

,

L ein Brückenglied,

A $C_2$-$C_8$-Alkylen, das gegebenenfalls durch 1 bis 3 Sauerstoffatome in Etherfunktion, Iminogruppen oder $C_1$-$C_4$-Alkyliminogruppen unterbrochen ist,

Y Vinyl oder einen Rest der Formel -$CH_2$-$CH_2$-Q, wobei Q für eine unter alkalischen Reaktionsbedingungen abspaltbare Gruppe steht, und

D Phenyl oder Naphthyl, wobei diese Reste ein- oder mehrfach durch Hydroxysulfonyl, Carboxyl, $C_1$-$C_4$-Alkoxycarbonyl, Cyano, $C_1$-$C_4$-Alkyl, $C_1$-$C_4$-Alkoxy, Halogen, Nitro oder Vinylsulfonyl substituiert sein können, bedeuten,

sowie ein Verfahren zur Herstellung von Reaktivfarbstoffen durch Oxidation der obengenannten Verbindungen.

Die EP-A-520 241 beschreibt die Herstellung von Azoreaktivfarbstoffen, wobei man von Azoverbindungen ausgeht, die eine 2-Hydroxyethylthioethergruppe aufweisen, diese in die 2-Hydroxyethylsulfonylgruppe überführt und daraus anschließend einen Schwefelsäurehalbester erzeugt.

Aus der US-A-5 182 371 sind Reaktivfarbstoffe bekannt, denen das Molekülgerüst der obengenannten Verbindungen der Formel I zugrunde liegt, die jedoch anstelle der Thioether- eine Sulfonylgruppe aufweisen. Diese Farbstoffe zeichnen sich durch besonders günstige anwendungstechnische Eigenschaften, insbesondere hohe Lichtechtheit, hohe Farbstärke und gutes Fixierverhalten, aus.

Aufgabe der vorliegenden Erfindung war es nun, neue Zwischenprodukte bereitzustellen, die sich in vorteilhafter Weise zur Herstellung dieser Reaktivfarbstoffe eignen.

Demgemäß wurden die eingangs näher bezeichneten Verbindungen der Formel I gefunden.

Die neuen Verbindungen der Formel I sind im vorliegenden Fall in der Regel in Form der freien Säure angegeben. Selbstverständlich werden jedoch auch ihre Salze mit umfaßt.

Als Salze kommen dabei Metall- oder Ammoniumsalze in Betracht. Metallsalze sind insbesondere die Lithium-, Natrium- oder Kaliumsalze. Unter Ammoniumsalzen im erfindungsgemäßen Sinne sind solche Salze zu verstehen, die entweder unsubstituierte oder substituierte Ammoniumkationen aufweisen. Substituierte Ammoniumkationen sind z.B. Monoalkyl-, Dialkyl-, Trialkyl-, Tetraalkyl- oder Benzyltrialkylamnoniumkationen oder solche Kationen, die sich von stickstoffhaltigen fünf- oder sechsgliedrigen gesättigten Heterocyclen ableiten, wie Pyrrolidinium-, Piperidinium-, Morpholinium-, Piperazinium- oder N-Alkylpiperaziniumkationen oder deren N-monoalkyl- oder N,N-dialkylsubstituierte Produkte. Unter Alkyl ist dabei im allgemeinen

geradkettiges oder verzweigtes $C_1$-$C_{20}$-Alkyl zu verstehen, das durch Hydroxylgruppen substituiert und/oder durch Sauerstoffatome in Etherfunktion unterbrochen sein kann.

Wenn in der obengenannten Formel I substituierte Phenylreste auftreten, so können dabei, sofern nicht anders vermerkt, z.B. $C_1$-$C_4$-Alkyl, $C_1$-$C_4$-Halogenalkyl, $C_1$-$C_4$-Alkoxy, Amino, $C_1$-$C_4$-Mono- oder Dialkylamino, Nitro, Formyl, Cyano, Carboxyl oder Hydroxysulfonyl als Substituenten in Betracht kommen. Sie weisen in der Regel 1 bis 3 Substituenten auf.

Reste $R^1$, $R^2$ und $R^3$ sind z.B. Methyl, Ethyl, Propyl, Isopropyl, Butyl, Isobutyl oder sec-Butyl.

Reste $R^4$ sind z.B. Methylsulfonyl, Ethylsulfonyl, Propylsulfonyl, Isopropylsulfonyl oder Butylsulfonyl.

Reste A sind z.B. $-(CH_2)_2-$, $-(CH_2)_3-$, $-(CH_2)_4-$, $-(CH_2)_5-$,$-(CH_2)_6-$, $-(CH_2)_7-$, $-(CH_2)_8-$, $-CH(CH_3)-CH_2-$, $-CH(CH_3)-CH(CH_3)-$, $-(CH_2)_2-O-(CH_2)_2-$, $-(CH_2)_2-NH-(CH_2)_2-$, $-(CH_2)_2-N(CH_3)-(CH_2)_2-$, $-(CH_2)_2-O-(CH_2)_2-O-(CH_2)_2-$ oder $-(CH_2)_2-O-(CH_2)_2-O-(CH_2)_2-O-(CH_2)_2-$.

Y in Formel I steht u.a. für eine unter alkalischen Reaktionsbedindungen abspaltbare Gruppe. Solche Gruppen sind z.B. Chlor, $OSO_3H$, $SSO_3H$, $OP(O)(OH)_2$, $C_1$-$C_4$-Alkylsulfonyloxy, gegebenenfalls substituiertes Phenylsulfonyloxy, $C_1$-$C_4$-Alkanoyloxy, $C_1$-$C_4$-Dialkylamino,

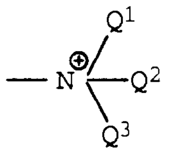 An⊖,

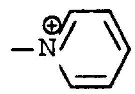 An⊖,

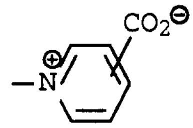 oder

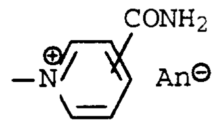 An⊖,

wobei $Q^1$, $Q^2$ und $Q^3$ gleich oder verschieden sind und unabhängig voneinander jeweils die Bedeutung von $C_1$-$C_4$-Alkyl oder Benzyl und $An^{\ominus}$ jeweils die Bedeutung eines Anions besitzen. (Geeignete Anionen sind z.B. Fluorid, Chlorid, Bromid, Iodid, Mono-, Di- oder Trichloracetat, Methylsulfonat, Phenylsulfonat oder 2- oder 4-Methylphenylsulfonat.)

Bevorzugte Gruppen Y sind $C_1$-$C_4$-Alkanoyloxy, insbesondere Acetyloxy.

Geeignete Brückenglieder L gehorchen z.B. der Formel

—NH—L$^1$—NH— , 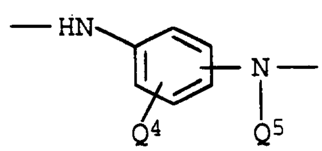 ,

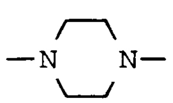 , 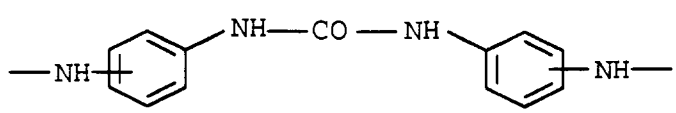 ,

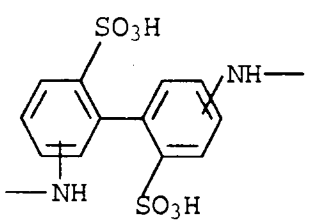 oder 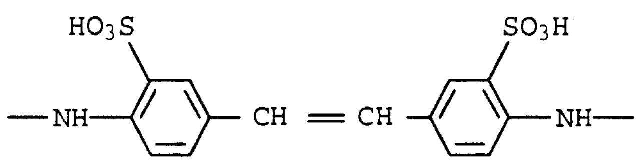 ,

wobei $L^1$ $C_2$-$C_8$-Alkylen, $Q^4$ Wasserstoff oder Hydroxysulfonyl und $Q^5$ Wasserstoff oder $C_1$-$C_4$-Alkyl bedeuten.

Einzelne Brückenglieder sind z.B. Reste der Formel

$—NH—C_2H_4—NH—$, $—NH—C_3H_6—NH—$, 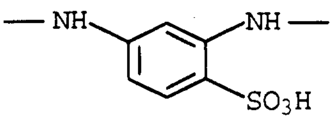 ,

oder 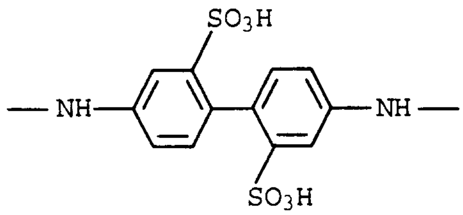 .

Reste D sind z.B. 2,4-Dihydroxysulfonylphenyl, 2,5-Dihydroxysulfonylphenyl, 2,5-Dihydroxysulfonyl-4-methylphenyl, 2,5-Dihydroxysulfonyl-6-chlorphenyl, 3,6,8-Trihydroxysulfonylnaphth-2-yl, 4,6,8-Trihydroxysulfonylnaphth-2-yl, 1,5-Dihydroxysulfonylnaphth-2-yl oder 1,6-Dihydroxysulfonylnaphth-2-yl.

Bevorzugt sind Verbindungen der Formel I, in der

| | |
|---|---|
| $R^1$, $R^2$ und $R^3$ | jeweils Wasserstoff, |
| $R^4$ | Fluor oder Chlor, |
| A | $C_2$-$C_4$-Alkylen, das gegebenenfalls durch ein Sauerstoffatom in Etherfunktion unterbrochen ist, und |
| D | Phenyl oder Naphthyl, wobei diese Reste durch Hydroxysulfonyl ein- bis dreifach substituiert sind, bedeuten. |

Insbesondere bevorzugt sind Verbindungen der Formel I, in der

D Dihydroxysulfonylphenyl oder Trihydroxysulfonylnaphthyl bedeutet.

Insbesondere bevorzugt sind weiterhin Verbindungen der Formel I, in der, wenn n 1 bedeutet, L für einen Rest der Formel

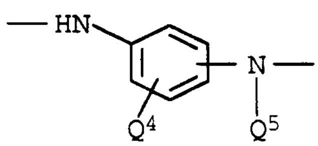

steht, in der $Q^4$ und $Q^5$ jeweils die obengenannte Bedeutung besitzen.

Die neuen Verbindungen der Formel I werden in vorteilhafter Weise erhalten, wenn man auf an sich bekanntem Weg z.B. ein Amin der Formel III

$D—NH_2$ (III),

in der D die obengenannte Bedeutung besitzt, diazotiert und mit einem Phenylendiamin der Formel IV

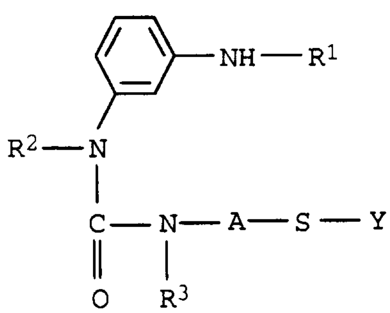 (IV),

in der $R^1$, $R^2$, $R^3$, A und Y jeweils die obengenannte Bedeutung besitzen, kuppelt.

Die resultierende Azoverbindung der Formel V

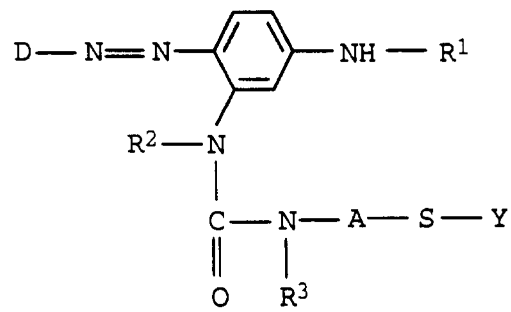

(V),

in der D, $R^1$, $R^2$, $R^3$, A und Y jeweils die obengenannte Bedeutung besitzen, kann dann in bekannter Weise mit Trihalogentriazin umgesetzt werden. Die Umsetzung kann dabei im Eintopfverfahren oder mit Isolierung der Zwischenprodukte durchgeführt werden.

Um zu denjenigen Verbindungen der Formel I zu gelangen, in der n null bedeutet, kann das Reaktionsprodukt mit weiterer Azoverbindung V umgesetzt werden.

Um zu denjenigen Verbindungen der Formel I zu gelangen, in der n eins bedeutet, kann das bei der Reaktion mit Trihalogentriazin entstehende Produkt mit einem Diamin der Formel VI

$H_2N—L—NH_2$ (VI),

in der L die obengenannte Bedeutung besitzt, umgesetzt werden.

Die Phenylendiamine der Formel IV können nach der in der älteren europäischen Patentanmeldung Nr. 93115520.4 beschriebenen Methode erhalten werden.

Die neuen Verbindungen der Formel I sind wertvolle Zwischenprodukte zur Herstellung von Reaktivfarbstoffen.

Demgemäß betrifft die vorliegende Erfindung weiterhin ein Verfahren zur Herstellung von Reaktivfarbstoffen der Formel II

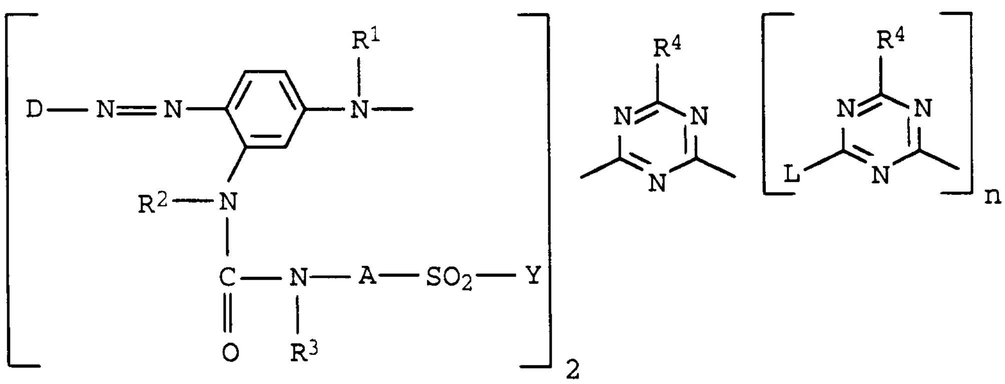

(II),

in der

| | |
|---|---|
| n | 0 oder 1 |
| $R^1$, $R^2$ und $R^3$ | gleich oder verschieden sind und unabhängig voneinander jeweils Wasserstoff, $C_1$-$C_4$-Alkyl oder Phenyl, |
| $R^4$ | Fluor, Chlor, Brom, $C_1$-$C_4$-Alkylsulfonyl, Phenylsulfonyl oder einen Rest der Formel 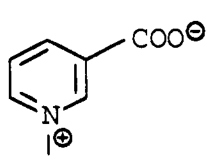 , |
| L | ein Brückenglied, |

A $C_2$-$C_8$-Alkylen, das gegebenenfalls durch 1 bis 3 Sauerstoffatome in Etherfunktion, Iminogruppen oder $C_1$-$C_4$-Alkyliminogruppen unterbrochen ist,

Y Vinyl oder einen Rest der Formel -$CH_2$-$CH_2$-Q, wobei Q für eine unter alkalischen Reaktionsbedingungen abspaltbare Gruppe steht, und

D Phenyl oder Naphthyl, wobei diese Reste ein- oder mehrfach durch Hydroxysulfonyl, Carboxyl, $C_1$-$C_4$-Alkoxycarbonyl, Cyano, $C_1$-$C_4$-Alkyl, $C_1$-$C_4$-Alkoxy, Halogen, Nitro oder Vinylsulfonyl substituiert sein können, bedeuten,

das dadurch gekennzeichnet ist, daß man Verbindungen der Formel I

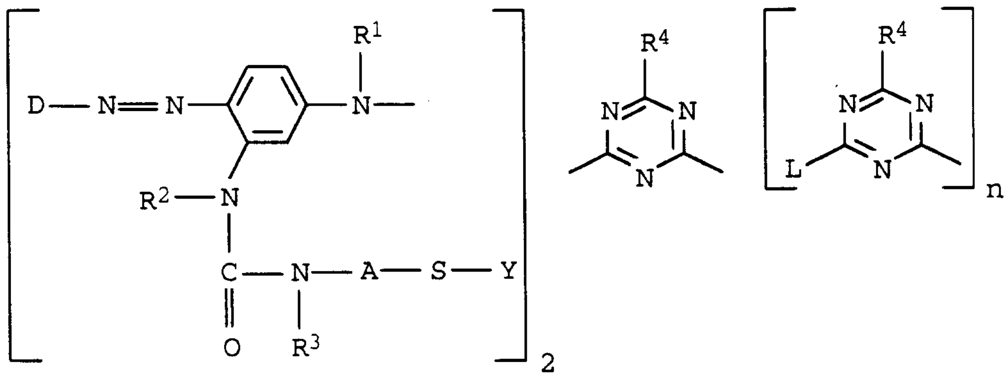

(I),

in der n, $R^1$, $R^2$, $R^3$, $R^4$, L, A, Y und D jeweils die obengenannte Bedeutung besitzen, in wäßrigem Medium mit einem Oxidationsmittel, gegebenenfalls in Anwesenheit von Katalysatoren, bei einer Temperatur von 0 bis 110 °C behandelt.

Was die beispielhafte Erläuterung der genannten Reste betrifft, sei auf die voranstehend vorgenommenen Ausführungen verwiesen.

Geeignete Oxidationsmittel sind organische oder anorganische Oxidationsmittel, wie Wasserstoffperoxid, Wasserstoffperoxid freisetzende Verbindungen, organische Hydroperoxide, Percarbonsäuren, Peroxomonoschwefelsäure oder deren Salze, insbesondere deren Alkalisalze, Peroxodischwefelsäure oder deren Salze, insbesondere deren Alkalisalze, Alkalihypohalogenite, Alkalipermanganate, Chromsäure oder deren Salze, insbesondere deren Alkalisalze, oder Salpetersäure.

Als Wasserstoffperoxid freisetzende Verbindungen können z.B. Alkaliperborate oder -percarbonate in Betracht kommen.

Geeignete organische Hydroperoxide sind z.B. Cumolhydroperoxid oder Alkylhydroperoxide, dabei insbesondere tert-Butylhydroperoxid.

Geeignete Percarbonsäuren sind z.B. Peressigsäure, m-Chlorperbenzoesäure, Magnesium-bis-(monoperoxyphthalat)-hexahydrat oder 1,12-Dodecandipersäure.

Die Verwendung von Wasserstoffperoxid als Oxidationsmittel ist bevorzugt.

Wasserstoffperoxid kommt im erfindungsgemäßen Verfahren in der Regel als 3 bis 80 gew.-%ige wäßrige Wasserstoffperoxidlösung zur Anwendung, wobei je mol Verbindung der Formel I im allgemeinen 2 bis 10 mol, vorzugsweise 2 bis 4 mol, wäßrige Wasserstoffperoxidlösung verwendet werden.

Bezogen auf einen Gewichtsteil der Verbindung der Formel I verwendet man üblicherweise weiterhin noch 1 bis 10 Gewichtsteile Wasser als Reaktionsmedium.

Das erfindungsgemäße Verfahren kann in Gegenwart von Katalysatoren durchgeführt werden. Geeignete Katalysatoren sind z.B. Alkaliwolframate, Alkalimolybdate oder Alkalivanadate, wie Lithium-, Natrium- oder Kaliumwolframat, -molybdat oder -vanadat.

Je Gewichtsteil Verbindung der Formel I verwendet man in der Regel 0,001 bis 0,1 Gewichtsteile an Katalysator.

Die Durchführung des neuen Verfahrens in Gegenwart eines Katalysators ist bevorzugt, wobei Alkaliwolframate, insbesondere Natriumwolframat, als Katalysatoren besonders hervorzuheben sind.

Erfindungsgemäß findet die Oxidation des Thioethers bei einer Temperatur von 0 bis 110 °C, vorzugsweise 20 bis 80 °C statt. Üblicherweise arbeitet man dabei unter atmosphärischem Druck.

Es empfiehlt sich, die Oxidation bei einem pH-Wert von 0 bis 8, vorzugsweise 4 bis 7, vorzunehmen.

Das neue Verfahren, das sowohl in kontinuierlicher als auch diskontinuierlicher Arbeitsweise vorgenommen werden kann, wird zweckmäßig so durchgeführt, daß man die Verbindung der Formel I zusammen mit Wasser und gegebenenfalls Katalysator vorlegt und gegebenenfalls unter Zugabe von Base, z.B. Natriumhydrogencarbonat, den obengenannten pH-Bereich einstellt. Dann erfolgt bei der obengenannten Temperatur die Zugabe des Oxidationsmittels. Anschließend wird im allgemeinen 0,5 bis 5 Stunden bei einer Temperatur von 20 bis 80 °C nachgerührt. Das resultierende Reaktionsgemisch wird dann filtriert und das den Reaktivfarbstoff II enthaltende Filtrat kann vom Lösungsmittel befreit und getrocknet werden.

Das neue Verfahren ist auf einfache Weise durchführbar und liefert die Reaktivfarbstoffe der Formel II in hoher Ausbeute und Reinheit.

Wie bereits oben ausgeführt, handelt es sich bei den Reaktivfarbstoffen der Formel II um wertvolle Produkte zum Färben oder Bedrucken von Hydroxygruppen oder Stickstoffatome aufweisenden organischen Substraten.

Die folgenden Beispiele sollen die Erfindung näher erläutern:

Beispiel 1

a) 462 g eines wasserfeuchten Nutschgutes (Trockengehalt: 70 Gew.-%) von Nitrophenylharnstoff wurden mit 700 g Xylol (Isomerengemisch) und 242 g 2-Aminoethyl-2'-hydroxyethylsulfid versetzt und zum Sieden erhitzt. Das bei 95 °C siedende Xylol-Wasser-Azeotrop wurde abdestilliert und anschließend das Reaktionsgemisch zur Entfernung des restlichen Xylols auf 140 °C erhitzt. Nach Beendigung der Umsetzung (DC-Kontrolle) wurde auf 50 °C abgekühlt und 1 Liter Wasser zugegeben. Das Reaktionsgemisch wurde mit 20 gew.-%iger Natronlauge auf einen pH-Wert von 11 gestellt. Anschließend wurde die wäßrige Phase abgetrennt und das in der organischen Phase verbliebene Wasser azeotrop abdestilliert. Nach Abkühlen auf 65 °C wurden 6 g 1-Methylimidazol zugegeben. Bei dieser Temperatur wurden 277 g Acetanhydrid zugetropft. Nach Abkühlen auf 20 °C saugte man den resultierenden Niederschlag ab. Man erhielt 555 g der Verbindung der Formel

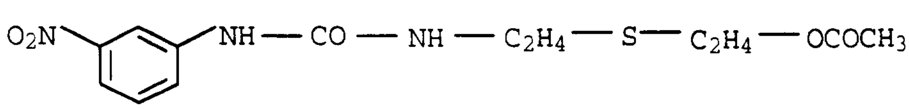 (Z1)

In analoger Weise können die folgenden Nitrophenylharnstoffe hergestellt werden:

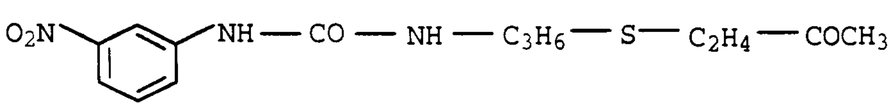 (Z2)

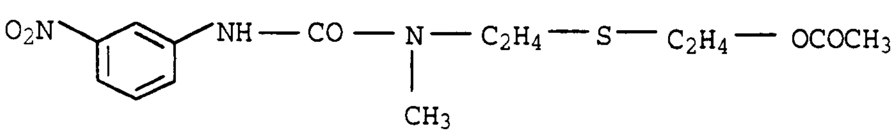 (Z3)

b) 565 g der Verbindung Z1 wurden in 1800 ml Isobutanol suspendiert. Hierzu wurden 130 g Raney-Nickel gegeben. Bei 55 bis 60 °C wurde dann innerhalb von 15 h durch Begasung mit Wasserstoff hydriert. Nach Beendigung der Reaktion (DC-Kontrolle) wurde vom Katalysator abfiltriert. Das Filtrat wurde unter vermindertem Druck eingeengt. Das verbleibende Öl kristallisierte nach kurzer Zeit durch. Die Kristalle wurden abgesaugt. Man erhielt 495 g der Verbindung der Formel

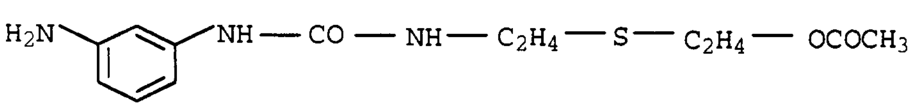 (Z4)

In analoger Weise können die folgenden Aminoverbindungen hergestellt werden:

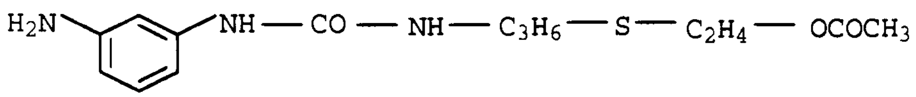

(Z5)

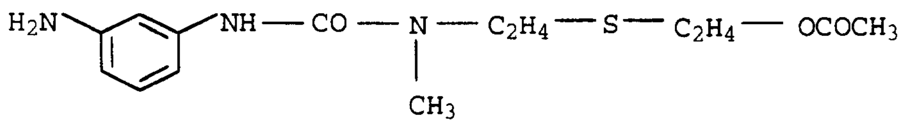

(Z6)

c) 101 g (0,4 mol) Anilin-2,4-disulfonsäure wurden in 400 ml Wasser salzsaurer diazotiert und zu einer Suspension aus 119 g (0,4 mol) der Verbindung Z4 in 500 ml Wasser gegeben. Die Kupplungsreaktion wurde bei 0 bis 5 °C und einem pH-Wert von 3 durch Zugabe von 2N Kaliumhydrogencarbonat-Lösung durchgeführt.

d) Die oben beschriebene Reaktionslösung wurde halbiert und die erste Hälfte mit 40,6 g (0,22 mol) Cyanurchlorid bei 0 bis 5 °C und einem pH-Wert von 5,5 kondensiert. Anschließend wurde die Lösung klärfiltriert und mit der zweiten Hälfte der Kupplungslösung bei 40 bis 50 °C und einem pH-Wert von 5,5 umgesetzt. Nach Aussalzen erhielt man 204 g der Verbindung

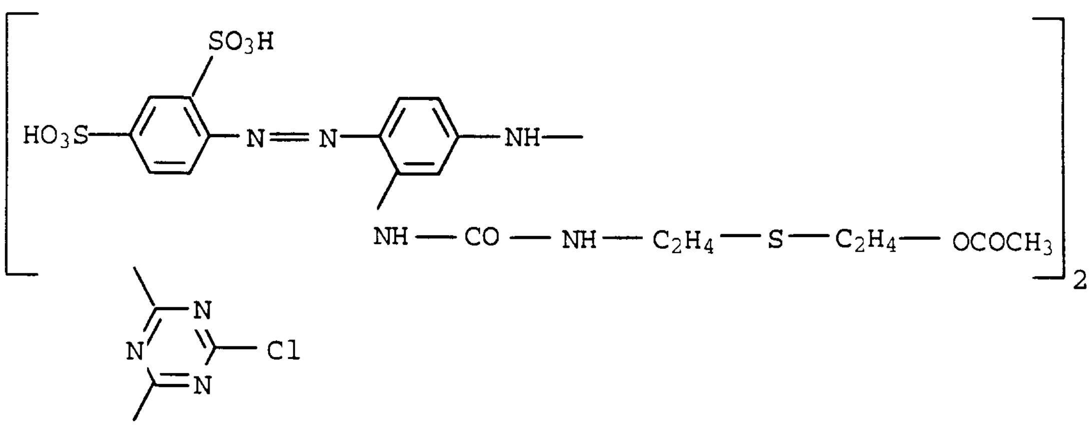

($\lambda_{max}$ = 378 nm)

Um die unter d) beschriebene Verbindung zu erhalten, kann auch wie folgt verfahren werden.

e) 254 g (1 mol) Anilin-2,4-disulfonsäure wurden in 1000 g Eis/Wasser-Gemisch bei 0 bis 5 °C mit 320 ml 23 gew.-%iger wäßriger Natriumnitritlösung diazotiert. Anschließend wurden 297 g (1,0 mol) der Verbindung Z4, in 500 g Wasser suspendiert, zugegeben und die Kupplungsreaktion mit Hilfe von wäßriger 1 N Natriumhydrogencarbonatlösung bei einem pH-Wert von 3 durchgeführt. Die entstandene Kupplungslösung wurde daraufhin mit einem Gemisch aus 92 g (0,5 mol) Cyanurchlorid und 250 g Eiswasser versetzt. Dann wurde bei 0 bis 5 °C und einem pH-Wert von 5,5, der durch Zugabe von Natriumhydrogencarbonatlösung eingestellt wurde, der 1. Kondensationsschritt durchgeführt. Nach 1 Stunde wurde das Reaktionsgemisch für den 2. Kondensationsschritt auf 40 bis 45 °C erwärmt und ein pH-Wert von 5,5 mittels Natriumhydrogencarbonatlösung eingehalten. Die resultierende Verbindung entspricht als freie Säure der in Beispiel 1d) beschriebenen Verbindung und wurde durch Aussalzen isoliert.

In analoger Weise können die in Tabelle 1 aufgeführten Verbindungen der Formel

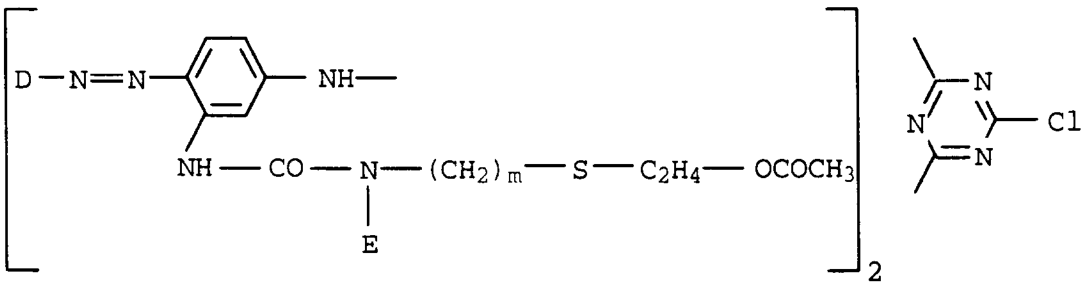

erhalten werden.

Tabelle 1

| Bsp. Nr. | D | E | m |
|---|---|---|---|
| 2 | 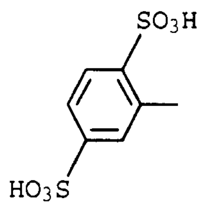 | H | 2 |
| 3 | 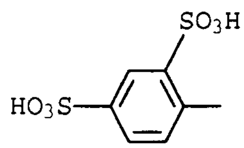 | H | 3 |
| 4 | 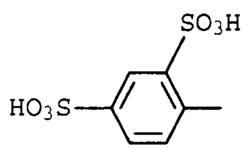 | $CH_3$ | 2 |
| 5 | 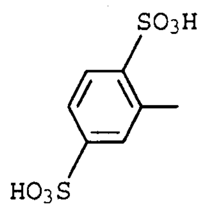 | H | 3 |
| 6 | 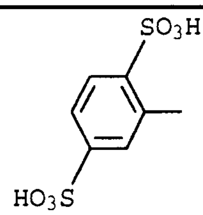 | $CH_3$ | 2 |
| 7 | 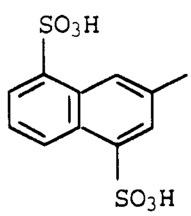 | H | 2 |
| 8 | 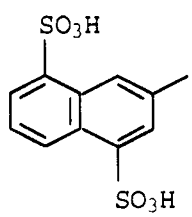 | H | 3 |

| Bsp. Nr. | D | E | m |
|---|---|---|---|
| 9 | 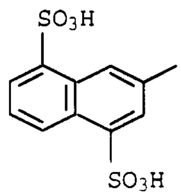 | $CH_3$ | 2 |
| 10 | 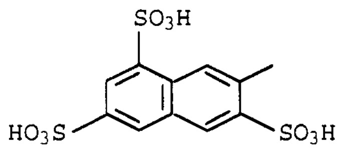 | H | 2 |
| 11 | 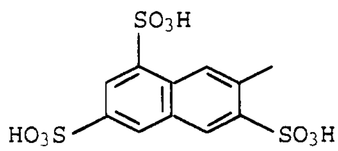 | H | 3 |
| 12 | 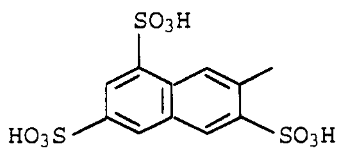 | $CH_3$ | 2 |
| 13 | 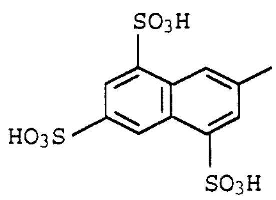 | $CH_3$ | 2 |
| 14 | 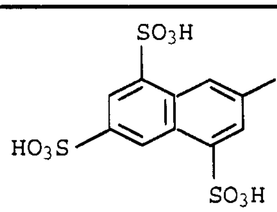 | H | 3 |
| 15 | 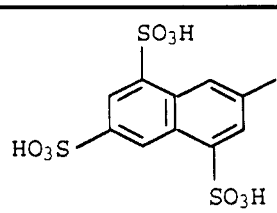 | $CH_3$ | 2 |
| 16 | 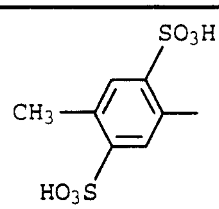 | H | 2 |

| Bsp. Nr. | D | E | m |
|---|---|---|---|
| 17 | 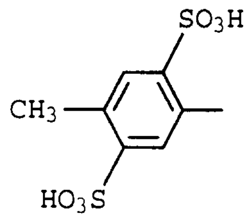 | H | 3 |
| 18 | 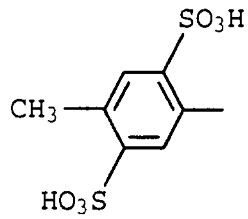 | $CH_3$ | 2 |
| 19 | 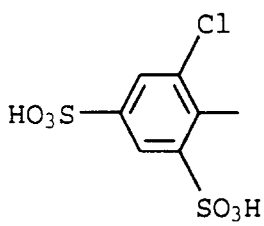 | H | 2 |
| 20 | 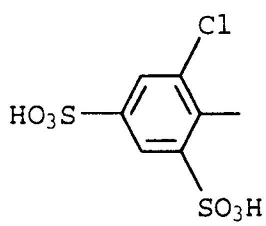 | H | 3 |
| 21 | 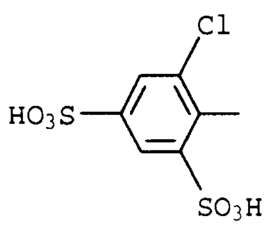 | $CH_3$ | 2 |

Beispiel 22

0,4 mol der in Beispiel 1c) beschriebenen Kupplungslösung wurden mit 81,2 g (0,44 mol) Cyanurchlorid bei 0 bis 5 ° C und einem pH-Wert von 5,5 kondensiert. Die Reaktionslösung wurde anschließend klärfiltriert und mit 21,6 g (0,2 mol) para-Phenylendiamin versetzt. Zur vollständigen Umsetzung wurde das Reaktionsgemisch auf 40 bis 50 ° C erwärmt und durch Zugabe von 2N Natronlauge ein pH-Wert von 5,5 eingehalten. Die Verbindung konnte durch Sprühtrocknen isoliert werden und entspricht der Formel

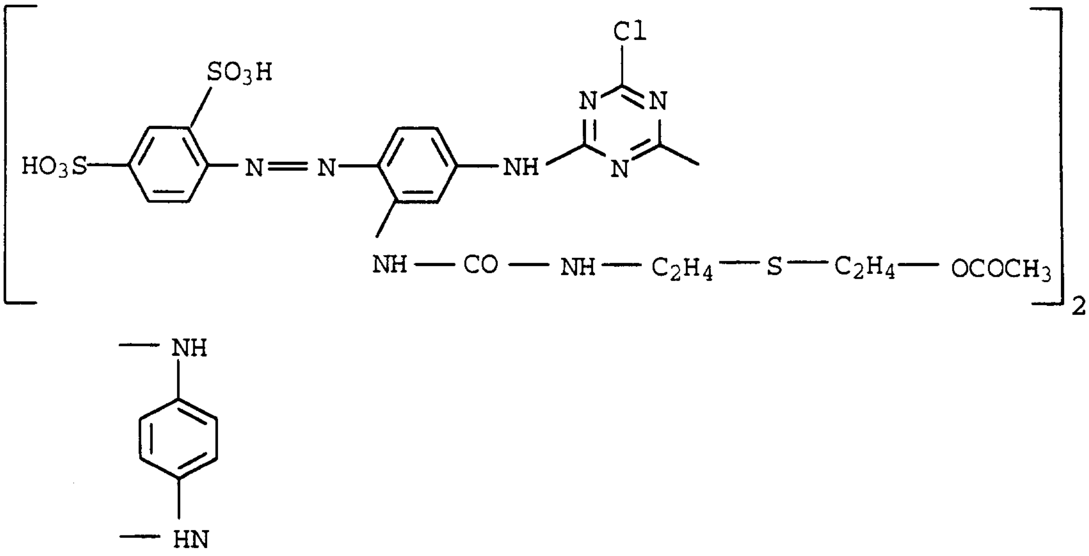

($\lambda_{max}$ = 391 nm)

In analoger Weise können die in Tabelle 2 aufgeführten Verbindungen der Formel

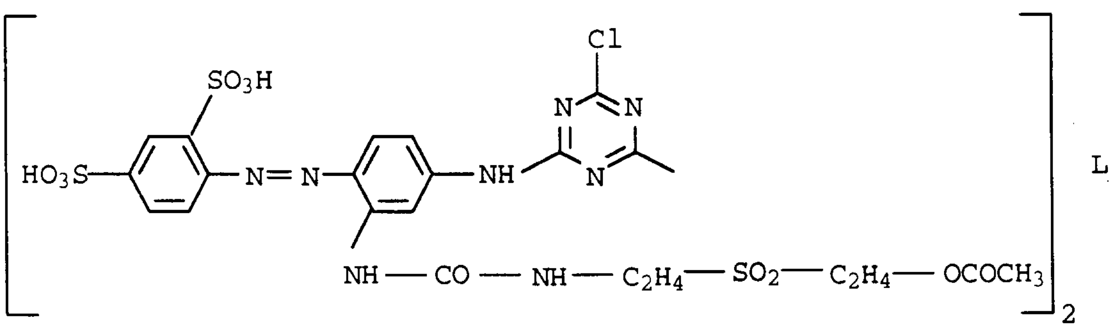

erhalten werden.

Tabelle 2

| Bsp. Nr. | L |
|---|---|
| 23 | 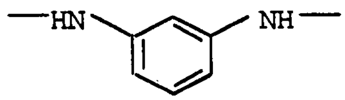 |
| 24 | $—HN— C_2H_4 — NH—$ |
| 25 | $—HN— C_2H_4 — NH—$ |
| 26 | 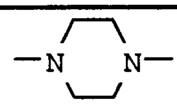 |
| 27 | 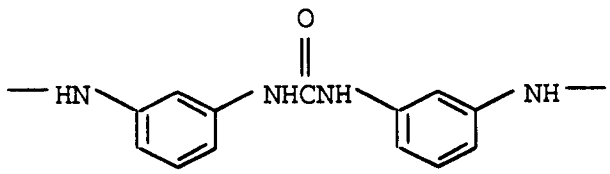 |

Beispiel 28

0,4 mol der in Beispiel 1c) beschriebenen Kupplungslösung wurden mit 81,2 g (0,44 mol) Cyanurchlorid bei 0 bis 5 °C und einem pH-Wert von 5,5 umgesetzt. Die Reaktionslösung wurde anschließend klärfiltriert und mit 37,6 g (0,2 mol) 2,4-Diaminobenzolsulfonsäure versetzt. Zur vollständigen Umsetzung wurde das Reaktionsgemisch auf 40 bis 50 °C erwärmt und mit 2N Natronlauge ein pH-Wert von 5,5 eingehalten. Die resultierende Verbindung wurde durch Eindampfen der Reaktionslösung isoliert. Sie entspricht der Formel

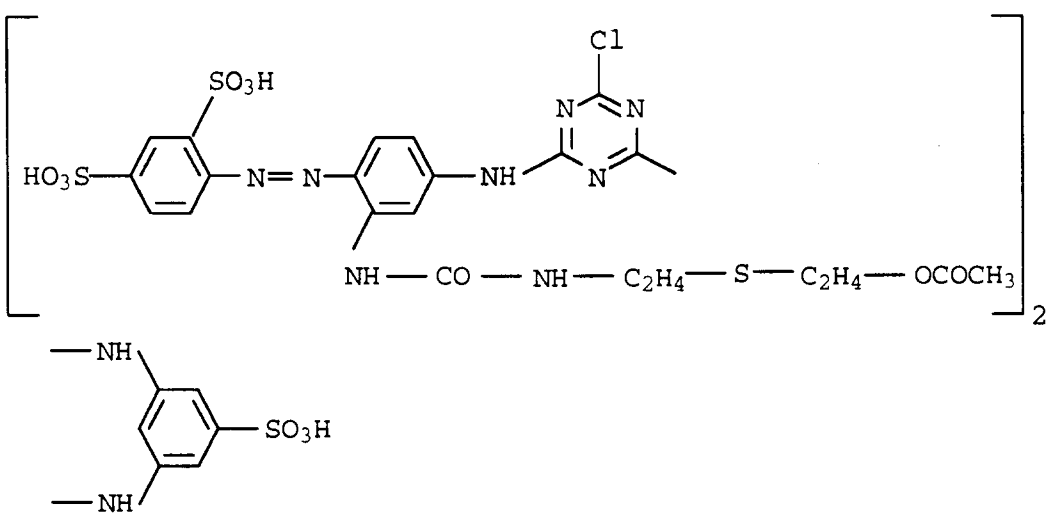

In analoger Weise können die in Tabelle 3 aufgeführten Verbindungen der Formel

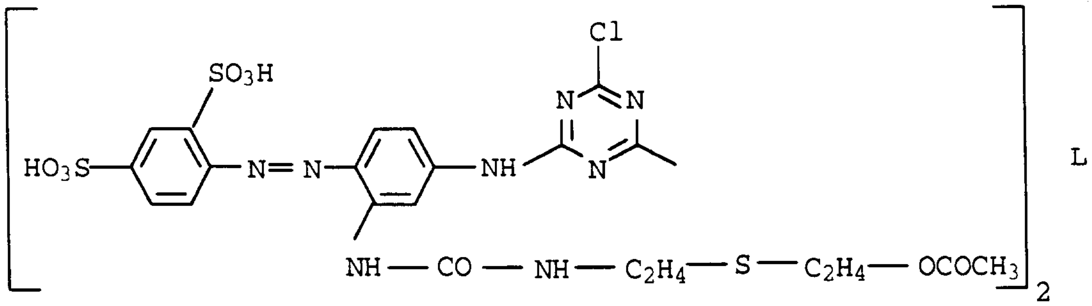

erhalten werden.

Tabelle 3

| Bsp. Nr. | L |
|---|---|
| 29 | 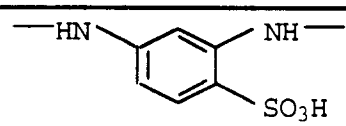 |
| 30 | 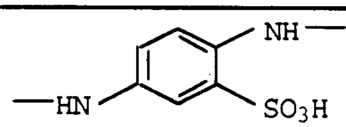 |
| 31 | 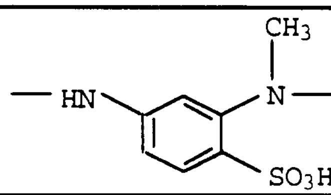 |
| 32 | 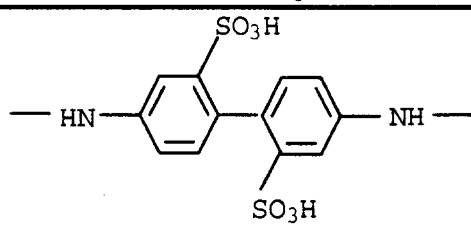 |
| 33 | 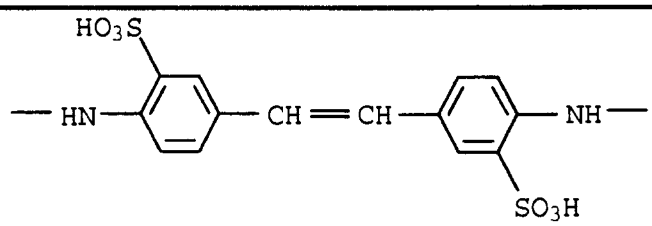 |

## Beispiel 34

100 g der nach Beispiel 1d) erhaltenen Verbindung wurden in 250 ml Wasser gegeben. Mit Natriumhydrogencarbonat wurde ein pH-Wert von 6 eingestellt. Dann wurden 0,5 g Natriumwolframat zugegeben und 20 g 30 gew.-%ige wäßrige Wasserstoffperoxidlösung zugetropft. Man rührte 2 Stunden bei 60 ° C nach und filtrierte das resultierende Reaktionsgemisch. Das Filtrat wurde unter vermindertem Druck eingeengt und getrocknet. Man erhielt 98 g des Reaktivfarbstoffes der Formel

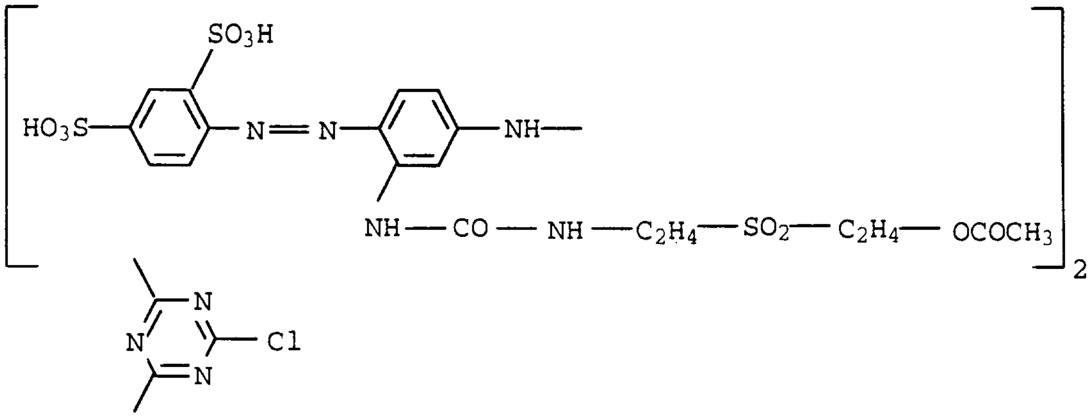

($\lambda_{max}$ = 378 nm)

Er färbt Baumwolle in gelbem Ton mit sehr guter Lichtechtheit und sehr guten Naßechtheiten.

In analoger Weise können die voranstehend aufgeführten Verbindungen zu den entsprechenden Reaktivfarbstoffen oxidiert werden.

**Patentansprüche**

**1.** Verbindungen der Formel I

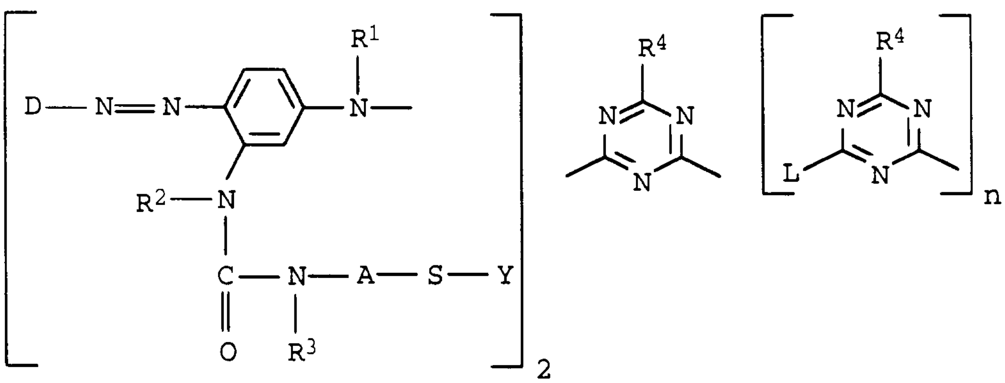

(I),

in der

| | |
|---|---|
| n | 0 oder 1 |
| $R^1$, $R^2$ und $R^3$ | gleich oder verschieden sind und unabhängig voneinander jeweils Wasserstoff, $C_1$-$C_4$-Alkyl oder Phenyl, |
| $R^4$ | Fluor, Chlor, Brom, $C_1$-$C_4$-Alkylsulfonyl, Phenylsulfonyl oder einen Rest der Formel |

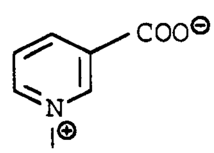

,

| | |
|---|---|
| L | ein Brückenglied, |
| A | $C_2$-$C_8$-Alkylen, das gegebenenfalls durch 1 bis 3 Sauerstoffatome in Etherfunktion, Iminogruppen oder $C_1$-$C_4$-Alkyliminogruppen unterbrochen ist, |

| | |
|---|---|
| Y | Vinyl oder einen Rest der Formel $-CH_2-CH_2-Q$, wobei Q für eine unter alkalischen Reaktionsbedingungen abspaltbare Gruppe steht, und |
| D | Phenyl oder Naphthyl, wobei diese Reste ein- oder mehrfach durch Hydroxysulfonyl, Carboxyl, $C_1$-$C_4$-Alkoxycarbonyl, Cyano, $C_1$-$C_4$-Alkyl, $C_1$-$C_4$-Alkoxy, Halogen, Nitro oder Vinylsulfonyl substituiert sein können, bedeuten. |

**2.** Verbindungen nach Anspruch 1, dadurch gekennzeichnet, daß

| | |
|---|---|
| $R^1$, $R^2$ und $R^3$ | jeweils Wasserstoff, |
| $R^4$ | Fluor oder Chlor, |
| A | $C_2$-$C_4$-Alkylen, das gegebenenfalls durch ein Sauerstoffatom in Etherfunktion unterbrochen ist, und |
| D | Phenyl oder Naphthyl, wobei diese Reste durch Hydroxysulfonyl ein- bis dreifach substituiert sind, bedeuten. |

**3.** Verfahren zur Herstellung von Reaktivfarbstoffen der Formel II

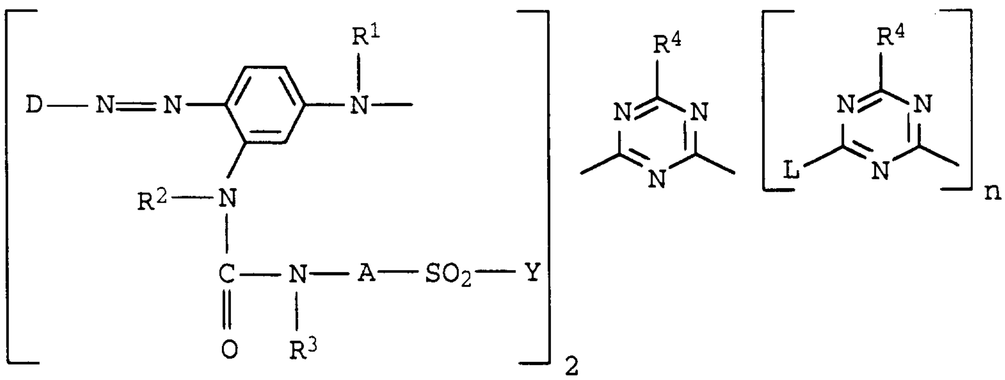

(II),

in der

| | |
|---|---|
| n | 0 oder 1 |
| $R^1$, $R^2$ und $R^3$ | gleich oder verschieden sind und unabhängig voneinander jeweils Wasserstoff, $C_1$-$C_4$-Alkyl oder Phenyl, |
| $R^4$ | Fluor, Chlor, Brom, $C_1$-$C_4$-Alkylsulfonyl, Phenylsulfonyl oder einen Rest der Formel |

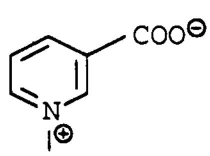

,

| | |
|---|---|
| L | ein Brückenglied, |
| A | $C_2$-$C_8$-Alkylen, das gegebenenfalls durch 1 bis 3 Sauerstoffatome in Etherfunktion, Iminogruppen oder $C_1$-$C_4$-Alkyliminogruppen unterbrochen ist, |
| Y | Vinyl oder einen Rest der Formel $-CH_2-CH_2-Q$, wobei Q für eine unter alkalischen Reaktionsbedingungen abspaltbare Gruppe steht, und |
| D | Phenyl oder Naphthyl, wobei diese Reste ein- oder mehrfach durch Hydroxysulfonyl, Carboxyl, $C_1$-$C_4$-Alkoxycarbonyl, Cyano, $C_1$-$C_4$-Alkyl, $C_1$-$C_4$-Alkoxy, Halogen, Nitro oder Vinylsulfonyl substituiert sein können, bedeuten, |

dadurch gekennzeichnet, daß man Verbindungen der Formel I

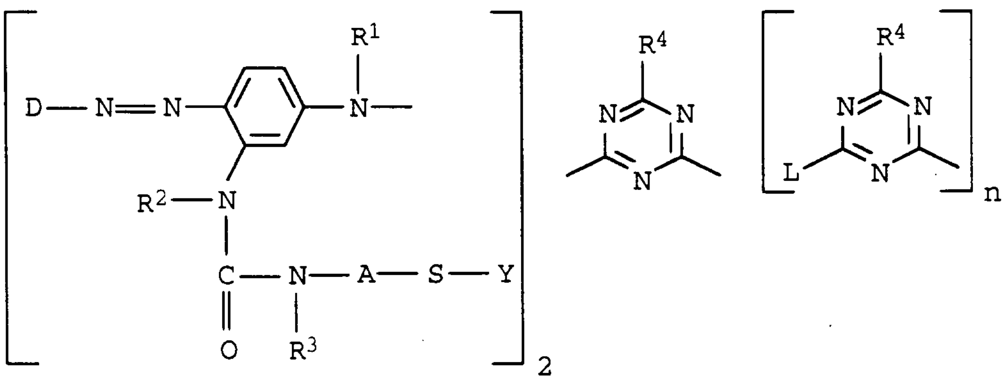

(I),

in der n, $R^1$, $R^2$, $R^3$, $R^4$, L, A, Y und D jeweils die obengenannte Bedeutung besitzen, in wäßrigem Medium mit einem Oxidationsmittel, gegebenenfalls in Anwesenheit von Katalysatoren, bei einer Temperatur von 0 bis 110 °C behandelt.

**4.** Verfahren nach Anspruch 3, dadurch gekennzeichnet, daß man als Oxidationsmittel Wasserstoffperoxid verwendet.

Europäisches Patentamt

# EUROPÄISCHER RECHERCHENBERICHT

Nummer der Anmeldung

EP 94108933.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| Y | EP - A - 0 503 385 (BASF) * Ansprüche 1,2 * & US-A-5 182 371 | 1-3 | C 09 B 62/507<br>C 09 B 62/08<br>C 07 D 251/30 |
| D,Y | EP - A - 0 520 241 (BAYER) * Zusammenfassung; Seite 10, Zeilen 9-20 * | 3,4 | |
| A | DE - A - 4 116 785 (BASF) * Zusammenfassung * | 1,4 | |
| A | EP - A - 0 437 699 (BASF) * Zusammenfassung * | 1,4 | |
| A | EP - A - 0 126 265 (BASF) * Zusammenfassung * | 1 | |

RECHERCHIERTE SACHGEBIETE (Int. Cl.⁵)

C 09 B
C 07 D 251/00

Der vorliegende Recherchenbericht wurde für alle Patentansprüche erstellt.

| Recherchenort | Abschlußdatum der Recherche | Prüfer |
|---|---|---|
| WIEN | 20-07-1994 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument